# EUROPEAN PATENT APPLICATION

(11) **EP 2 727 941 A1**
(43) Date of publication of application: **07.05.2014**
(21) Application number: 12007504.9
(22) Date of filing: 05.11.2012
(51) Int. Cl.: C07K 16/28, C07K 16/32, C07K 16/00

(54) **Method for the production of multispecific antibodies**

(71) Applicant: MAB Discovery GmbH, 82061 Neuried (DE)
(72) Inventor: Fischer, Stephan, 82362 Weilheim (DE); Renninger, Stephanie, 81379 München (DE)
(74) Representative: Schreiner, Siegfried

(57) **Abstract**

An antibody, characterized in specifically binding to three, four or five antigens selected from the group consisting of target antigens, preferably to human c-Met and IGF-1R or to c-Met and/or IGF-1R and at least one antigen selected from the group consisting of EGFR, HER2 and HER3, is useful for the manufacture of a pharmaceutical composition and especially for the treatment of breast, colon, lung, or pancreatic cancer.

SEQUENCE LISTING

<110> Mab Discovery GmbH

<120> Method for the production of multispecific antibodies

<160> 3

<170> BiSSAP 1.2

<210> 1
<211> 11
<212> PRT
<213> Oryctolagus

<400> 1
Trp Gly Pro Gly Thr Leu Val Thr Val Ser Ser
1               5                   10

<210> 2
<211> 11
<212> PRT
<213> Oryctolagus

<400> 2
Trp Gly Pro Gly Thr Leu Val Thr Val Ser Leu
1               5                   10

<210> 3
<211> 10
<212> PRT
<213> Oryctolagus

<400> 3
Phe Gly Gly Gly Thr Glu Val Val Val Lys
1               5                   10

## Description

The present invention relates to a method for the production of multispecific antibodies, specifically binding to two, three, four or five antigens selected from the group consisting of target antigens, especially therapeutically relevant antigens, e.g. mammalian, viral, bacterial or plant antigens, preferably from the group consisting of human c-Met and IGF-1R or c-Met and/or IGF-1R and at least one antigen selected from the group consisting of human EGFR, HER2 and HER3 compositions, methods for the production and uses thereof.

### Background of the invention

It is already known since nearly about 30 years that bispecific antibodies might be advantageous for the treatment of diseases (Staerz UD, et al., Nature (1985) 314(6012):628-31). According to WO1999064461 it was possible to generate homogeneous bispecific antibodies against Apo2 and DR4 by immunization of mice with mixed antigens. Bispecific antibodies are produced nowadays as heterogenic bispecific antibodies, consisting of variable chains of two different antibodies (see e.g. Chames P. and Baty D., MAbs. (2009) 1(6): 539-547). A further approach is the affinity maturation of an antibody which might result in some cases in cross reactive antibodies (e. g. WO2010108127, WO2011056997).

Human Epidermal growth factor receptor (EGFR, HER1), its sequence and functions are described by UniProt P00533 (EC=2.7.10.1), human HER2, its sequence and functions are described by UniProt P04626 (Receptor tyrosine-protein kinase erbB-2, EC=2.7.10.1), human HER3, its sequence and functions are described by UniProt P21860 (Receptor tyrosine-protein kinase erbB-3, EC=2.7.10.1). Fendry BM. et al., Cancer Res. 50 (1990 1550-1558 generated by immunization in mice with EGFR an anti-EGFR antibody which moderately binds also to p185^{HER2}. Bostrom J. et al., Science 323 (2009) 1610-1614 generated a repertoire of Herceptin^{®} antibody variants with mutations in the light chain (LC). Fabs thereof were generated which bind also VEGF. Bostrom J. et al. PLOS & (2011) e17887 discusses the thermodynamic background of mulitispecificity of antibodies. Schaefer G. et al., Cancer Cell 20 (2011) 472 generated an antibody with binding to HER3/EGFR by phage display. WO9964461 relates to bispecific antibodies against Apo2 and DR4. Immunization of mice was performed with mixed antigens. WO9939729 relates to the use of an HER2, HER3 and/or HER4 activating ligand which is a heregulin (HRG) polypeptide. WO2008027236 relates to a method of making a bispecific antibody against VEGF and HER2 by diversifying VL. WO2007146959 relates to dimeric and oligomeric structures of HER extracellular domains and HER/Fc molecules. WO2008140493 relates to anti-EGFR family member antibodies and heterogenic bispecific antibodies comprising anti-EGFR family member antibodies. WO2010129304 relates to methods for making heterogenic molecules, such as bispecific antibodies. WO2010108127 relates to a heterogenic bispecific antibody comprising an antigen-binding domain that specifically binds to EGFR and HER3 wherein the toxicity of the antibody is less than the toxicity of an EGFR antagonist. WO2011056997 relates to a method of affinity maturation of an antibody. WO2011060206 relates to an antibody against HER-3 and a combination thereof with other antibodies of the HER family. WO2011140254 relates to a combination of anti-EGFR antibodies. WO2012007167 relates to a heterogenic bispecific antibody against HER2 and LewisY. Heterogenic bispecific antibodies against c-Met and EGFR which comprise a first antigen binding site binding to c-Met and a second antigen binding site binding to EGFR are mentioned in WO2010115551 and WO2010034441. Further heterogenic bispecific antibodies against targets of the HER family, c-Met and IGF-1R are mentioned in WO2006091209.

C-Met (Hepatocyte growth factor receptor, HGF receptor, EC=2.7.10.1, P08581 UniProtKB/Swiss-Prot) is a proto-oncogene (see e.g. Dean, M., et al., Nature 318 (1985) 385-8; Chan, A.M., et al., Oncogene 1 (1987) 229-33; Bottaro, D.P., et al., Science 251 (1991) 802-4). Antibodies against c-Met are known e.g. from US5686292, US7476724, WO2004072117, WO2004108766, WO2005016382, WO2005063816, WO2006015371, WO2006104911, WO2007126799, and WO2009007427.

Insulin-like growth factor 1 receptor (IGF-1R, EC 2.7.112, CD 221 antigen) belongs to the family of transmembrane protein tyrosine kinases (LeRoith, D., et al., Endocrin. Rev. 16 (1995) 143-163; and Adams, T.E., et al., Cell. Mol. Life Sci. 57 (2000) 1050-1093). Antibodies against IGF-1R are for example mentioned in WO2007115814, WO2004087756, WO2005005635, WO2006008639, US20050249730, US20050084906, WO2005058967, WO2006013472, US20030165502, WO2005082415, WO2005016970, WO03106621, WO04083248, WO2003100008, WO2004087756, WO2005005635 and WO2005094376.

Methods for the production of monospecific antibodies e.g. from single cells are e.g. mentioned in WO2011147903, WO2007003041, WO2008045140, WO2004106377, EP1255780, and EP1633787.

However so far no method is known which is useful for providing homogeneous bi- and multispecific antibodies in a reproducible and easy manner.

### Summary of the Invention

The invention provides a method for the production of a monoclonal antibody specifically binding to two, three, four or five antigens selected from the group consisting of target antigens, characterized in performing in the order specified the steps of
i) immunizing a rabbit or chicken with two, three, four or five said antigens,
ii) isolating B cells from said rabbit or chicken,
iii) isolating B cells from said B cells isolated in step ii) which bind at least to one of said antigens,
iv) isolating single B cells from said B cells isolated in step iii),
v) selecting one of said single B cells which comprises mRNA encoding a polypeptide, comprising a VH region and/or a polypeptide comprising a VL region of an antibody which binds specifically to said two, three, four or five antigens, and
vi) producing the antibody expressed by said single B cell.

The invention provides a method for the production of a monoclonal antibody specifically binding to two, three, four or five antigens selected from the group consisting of therapeutically relevant antigens, characterized in performing in the order specified the steps of
i) immunizing a rabbit or chicken with two, three, four or five said antigens,
ii) isolating B cells from said rabbit or chicken,
iii) isolating B cells from said B cells isolated in step ii) which bind at least to one of said antigens,
iv) isolating single B cells from said B cells isolated in step iii),
v) selecting one of said single B cells which comprises mRNA encoding a polypeptide, comprising a VH region and/or a polypeptide comprising a VL region of an antibody which binds specifically to said two, three, four or five antigens, and
vi) producing the antibody expressed by said single B cell.

The invention provides a method for the production of a monoclonal antibody specifically binding to two, three, four or five antigens selected from the group consisting of viral antigens, characterized in performing in the order specified the steps of
i) immunizing a rabbit or chicken with two, three, four or five said antigens,
ii) isolating B cells from said rabbit or chicken,
iii) isolating B cells from said B cells isolated in step ii) which bind at least to one of said antigens,
iv) isolating single B cells from said B cells isolated in step iii),
v) selecting one of said single B cells which comprises mRNA encoding a polypeptide, comprising a VH region and/or a polypeptide comprising a VL region of an antibody which binds specifically to said two, three, four or five antigens, and
vi) producing the antibody expressed by said single B cell.

Preferably the invention provides a method for the production of a monoclonal antibody specifically binding to two, three, four or five antigens selected from the group consisting of mammalian antigens,
characterized in performing in the order specified the steps of
i) immunizing a rabbit or chicken with two, three, four or five said antigens,
ii) isolating B cells from said rabbit or chicken,
iii) isolating B cells from said B cells isolated in step ii) which bind at least to one of said antigens,
iv) isolating single B cells from said B cells isolated in step iii),
v) selecting one of said single B cells which comprises mRNA encoding a polypeptide, comprising a VH region and/or a polypeptide comprising a VL region of an antibody which binds specifically to said two, three, four or five antigens, and
vi) producing the antibody expressed by said single B cell.

Preferably the invention provides a method for the production of a monoclonal antibody specifically binding to two, three, four or five antigens selected from the group consisting of parasitic antigens,
characterized in performing in the order specified the steps of
i) immunizing a rabbit or chicken with two, three, four or five said antigens,
ii) isolating B cells from said rabbit or chicken,
iii) isolating B cells from said B cells isolated in step ii) which bind at least to one of said antigens,
iv) isolating single B cells from said B cells isolated in step iii),
v) selecting one of said single B cells which comprises mRNA encoding a polypeptide, comprising a VH region and/or a polypeptide comprising a VL region of an antibody which binds specifically to said two, three, four or five antigens, and
vi) producing the antibody expressed by said single B cell.

Preferably the invention provides a method for the production of a monoclonal antibody specifically binding to two, three , four or five antigens selected from the group consisting of bacterial antigens,
characterized in performing in the order specified the steps of
i) immunizing a rabbit or chicken with two, three, four or five said antigens,
ii) isolating B cells from said rabbit or chicken,
iii) isolating B cells from said B cells isolated in step ii) which bind at least to one of said antigens,
iv) isolating single B cells from said B cells isolated in step iii),
v) selecting one of said single B cells which comprises mRNA encoding a polypeptide, comprising a VH region and/or a polypeptide comprising a VL region of an antibody which binds specifically to said two, three, four or five antigens, and
vi) producing the antibody expressed by said single B cell.

Preferably the invention provides a method for the production of a monoclonal antibody specifically binding to two, three, four or five antigens selected from the group consisting of plant antigens,
characterized in performing in the order specified the steps of
i) immunizing a rabbit or chicken with two, three, four or five said antigens,
ii) isolating B cells from said rabbit or chicken,
iii) isolating B cells from said B cells isolated in step ii) which bind at least to one of said antigens,
iv) isolating single B cells from said B cells isolated in step iii),
v) selecting one of said single B cells which comprises mRNA encoding a polypeptide, comprising a VH region and/or a polypeptide comprising a VL region of an antibody which binds specifically to said two, three, four or five antigens, and
vi) producing the antibody expressed by said single B cell.

The invention provides a method for the production of a monoclonal antibody specifically binding to c-Met and IGF-1R or c-Met and/or IGF-1R and at least one antigen selected from the group consisting of EGFR, HER2 and HER3, characterized in performing in the order specified the steps of
i) immunizing a rabbit or chicken with antigens c-Met and IGF-1R or c-Met and/or IGF-1R and at least one antigen selected from the group consisting of EGFR, HER2 and HER3"
ii) isolating B cells from said rabbit or chicken,
iii) isolating B cells from said B cells isolated in step ii) which bind to human c-Met, IGF-1R, EGFR, HER2 and/or HER3,
iv) isolating single B cells from said B cells isolated in step iii),
v) selecting one of said single B cells which comprises mRNA encoding a polypeptide comprising a VH region and/or a polypeptide comprising a VL region of an antibody which binds specifically to c-Met and IGF-1R or to c-Met and/or IGF-1R and at least one antigen selected from the group consisting of EGFR, HER2 and HER3, and
vi) producing the antibody expressed by said single B cell.

Preferably the antigen used for immunization, preferably c-Met, IGF-1R, EGFR, HER2, and/or HER3 antigen is/are fusion polypeptides consisting of said antigen and a human Fc polypeptide. Preferably in step i) CFA is used as adjuvant. Preferably in step i) CFA and IFA are used together as adjuvants.

Preferably in step ii) B cells are isolated from the blood of the rabbit or chicken. B cells are isolated preferably as PBMCs and depleted from macrophages. The antigens used for isolating B cells in step iii) can be the target proteins, preferably c-Met, IGF-1R, EGFR, HER2, and/or HER3 proteins, fragments thereof, preferably the extracellular domain or parts thereof, cells presenting the antigens on their surface or the like.

Preferably in step iv) single B cells, secreting immunoglobulin, preferably IgG, are separated, preferably by FACS. Preferably the single B cell is then treated with a feeder cell before performing step v).

Preferably the antibody produced by the single B cell is tested, preferably by ELISA, whether it binds specifically to the respective antigens. Preferably the antibody is tested whether it binds specifically to c-Met and IGF-1R or to c-Met and/or IGF-1R and at least one antigen selected from the group consisting of EGFR, HER2 and HER3 (if immunization was performed with these antigens) and selected if it binds. Preferably the antibody is recombinantly produced based on its nucleic acid and/or polypeptide sequence.

The invention relates to multispecific monoclonal antibodies against human c-Met and IGF-1R or to c-Met and/or IGF-1R and at least one antigen selected from the group consisting of human EGFR, HER2 and HER3 and methods for the generation of such antibodies. Human c-Met, IGF-1R, EGFR, HER2 and HER3 are therapeutic targets.

The invention provides also compositions, B cells, methods of use, and methods of production of the antibodies according to the invention.

The invention comprises a monoclonal antibody, characterized in specifically binding to three, four or five antigens selected from the group of target antigens, preferably characterized in specifically binding to c-Met and IGF-1R or to c-Met and/or IGF-1R and at least one antigen selected from the group consisting of EGFR, HER2 and HER3. Preferably the antibody is a rabbit monoclonal antibody. The antibody according to the invention is preferably characterized in being a humanized or chimeric version of said antibody. Preferably, the antibody according to the invention is an antibody comprising antigen binding sequences from a rabbit or chicken donor grafted to a heterologous non-human, human, or humanized sequence (e.g., framework and/or constant domain sequences). Preferably, an antibody of the invention has rabbit or chicken V regions or rabbit or chicken CDR regions and a human C region and/or framework. Preferably, the rabbit or chicken VL region or a human framework region comprising rabbit or chicken light chain CDRs is fused to a human kappa light chain constant region. Preferably, the rabbit or chicken VH region or a human framework region comprising rabbit or chicken heavy chain CDRs is fused to a human constant region, preferably IgG1.

The invention also provides a pharmaceutical composition characterized by comprising an antibody according to the invention. The invention also provides the use of an antibody according to the invention for the manufacture of a pharmaceutical composition. The invention also provides an antibody according to the invention for the treatment of a patient in the need of such treatment, preferably in the treatment of cancer. The invention also provides an antibody according to the invention for the treatment of breast, colon, lung, or pancreatic cancer. The invention also provides the use of an antibody according to the invention for manufacture of a medicament for the treatment of a patient in the need of such treatment, preferably in the treatment of cancer. The invention also provides the use of an antibody according to the invention for manufacture of a medicament for the treatment of breast, colon, lung, or pancreatic cancer. The invention also provides an antibody according to the invention for use in the treatment of a patient in the need of such treatment, preferably in the treatment of cancer, preferably in the treatment ofbreast, colon, lung, or pancreatic cancer.

The invention also provides a nucleic acid encoding an antibody according to the invention. The invention also provides an expression vector characterized in comprising a nucleic acid according to the invention for the expression of an antibody according to the invention in a prokaryotic or eukaryotic host cell. The invention also provides a prokaryotic or eukaryotic host cell comprising a nucleic acid according to the invention. The invention also provides a method of producing an antibody according to the invention characterized by expressing a nucleic acid according to the invention in a prokaryotic or eukaryotic host cell and recovering said antibody from said cell or the cell culture supernatant.

Preferably the anti- c-Met and/or IGF-1R, EGFR, HER2, and HER3 antibodies of the present invention are antagonistic antibodies.

The invention also provides a method for inhibiting c-Met, IGF-1R, EGFR, HER2 and/or HER3 activity in a cell expressing c-Met, IGF-1R, EGFR, HER2 and/or HER3, comprising contacting the cell with an antibody according to the invention.

### Detailed Description of the Invention

The term "rabbit" according to the invention means animals of the family Leporidae, preferably of genus Oryctolagus. The term "chicken" according to the invention means animals of the family Gallus gallus, preferably Gallus gallus domesticus.

The term "antibody" encompasses the various forms of antibody structures including, but not being limited to, whole antibodies and antibody fragments. The antibody according to the invention is in its primary form produced by a B-cell of a rabbit or chicken and binds to the respective antigens. Preferably the antibody in its primary form binds specifically to human c-Met and IGF-1R or to c-Met and/or IGF-1R and at least one antigen selected from the group consisting of EGFR, HER2 and HER3. Therefore the antibody according to the invention binds specifically to human c-Met and IGF-1R or to c-Met and/or IGF-1R and at least one antigen selected from the group consisting of EGFR, HER2 and HER3 based on its antigen-binding portion, preferably its VH region comprising three VH CDRs and/or its VL region comprising three VL CDRs. The term "VL (or VH) region" has the same meaning as VL (or VH) domain. The antibody according to the invention is in its primary form a mature antibody, which may be different from a simple germline antibody. Without being bound by theory, it is believed that binding of the antigen to a germline antibody might lead to significant structural rearrangements, whereas the unbound state of a matured antibody might be closer to its bond state. Therefore the mature form of the antibody has probably a more rigid structure than the germline form. The germline antibody might be therefore more conformational flexible, resulting in a slower binding rate (see e.g. Wedemayer GJ et al., Science. 1997 Jun 13;276(5319):1665-9; Structural insights into the evolution of an antibody combining site). The presumably lower flexible structure of the mature antibody may improve the physicochemical properties of the antibody according to the invention, as being e.g. solubility or low aggregation, leading to improved therapeutic properties. The antibody according to the invention as identified from a rabbit B cell is an antibody having variable regions of natural origin. "Natural origin" means according to the invention, that such an antibody has variable regions which are identical in their amino acid sequences to the sequences of variable regions naturally occurring in rabbits. The antibody according to the invention can be further modified and is preferably a rabbit or chicken antibody, a humanized antibody, a chimeric antibody, a fragment thereof, or a further genetically engineered and recombinant produced antibody as long as the characteristic properties according to the invention are retained. The antibody can be bound to a further agent, e.g. as being an immunoconjugate.

The term "rabbit monoclonal antibody "means a monoclonal antibody produced by immunizing a rabbit and isolated from a B cell of said rabbit. Preferably the antibody is from a B cell of said rabbit. Preferably the rabbit antibody according to the invention is characterized in that it comprises at the C terminal end of the VH region SEQ NO:1 or 2 and/or at the C terminal end of the VL region SEQ ID NO:3.The term "chicken antibody" means an antibody produced by immunizing a chicken and isolated from a B cell of said chicken. Preferably the antibody is isolated from a B cell of said chicken.

An "immunoconjugate" means an antibody conjugated to one or more cytotoxic agents, such as a chemotherapeutic agent, a drug, a growth inhibitory agent, a toxin, another antibody or a radioactive isotope.

The term "target antigen" according to the invention means plant antigens and therapeutically relevant antigens or antigenic fragments thereof. The target antigen is preferably a protein, polypeptide or peptide antigen, nucleic acid antigen, carbohydrate antigen or a whole or attenuated or inactivated organism such as a bacterium, virus, parasite or protozoa. Preferably immunization is performed with two, three, four or five antigens of the same biological class, preferably order, preferably family, preferably genus, preferably species.

The term "therapeutically relevant antigen" according to the invention means a mammalian, viral, bacterial, protozoa or parasitic antigen. Preferably the antigen is a antigen presented on the surface of a mammalian or bacterial cell. Therapeutically relevant viruses are Adenoviridae, like Adenovirus, Herpesviridae, like Herpes simplex, type 1, Herpes simplex, type 2, Varicella-zoster virus, Epstein-barr virus, Human cytomegalovirus, Human herpesvirus, type 8, Papillomaviridae like Human papillomavirus, Polyomaviridae like BK virus, JC virus, Poxviridae, like Smallpox, Hepadnaviridae, like Hepatitis B virus, Parvoviridae, like Human bocavirus, Parvovirus B19, Astroviridae, like Human astrovirus, Caliciviridae like Norwalk virus, Picornaviridae like coxsackievirus, hepatitis A virus, poliovirus, rhinovirus, Coronaviridae like Severe acute respiratory syndrome virus, Flaviviridae like Hepatitis C virus, yellow fever virus, dengue virus, West Nile virus Togaviridae like Rubella virus, Retroviridae like Human immunodeficiency virus (HIV), Orthomyxoviridae like Influenza virus , Arenaviridae like Guanarito virus, Junin virus, Lassa virus, Machupo virus, Sabiá virus, Bunyaviridae like Crimean-Congo hemorrhagic fever virus, Filoviridae like Ebola virus, Marburg virus, Paramyxoviridae like Measles virus, Mumps virus, Parainfluenza virus, Respiratory syncytial virus, Human metapneumovirus, Rhabdoviridae like Rabies virus, Reoviridae like Rotavirus; and Hepatitis D and E virus.

Preferred viral antigens according to the invention are antigens derived from herpes simplex virus (HSV) types 1 and 2, such as HSV-1 and HSV-2 glycoproteins gB, gD and gH; antigens derived from varicella zoster virus (VZV), Epstein-Barr virus (EBV) and cytomegalovirus (CMV) including CMV gB and gH; and antigens derived from other human herpesviruses such as HHV6 and HHV7. (Chee et al., Cytomegaloviruses (J. K. McDougall, ed., Springer-Verlag 1990) ; McGeoch et al., J. Gen. Virol. (1988) 69:1531-1574, Baer et al., Nature (1984) 310:207-211; Davison and Scott, J. Gen. Virol. (1986) 67:1759-1816).

The term "bacterial antigen" according to the invention means such antigens as those derived from organisms that cause diphtheria, cholera, tuberculosis, tetanus, pertussis, meningitis, and other pathogenic states, preferably Bordetella pertussis, Neisseria meningitides (A, B, C, Y), Hemophilus influenza type B (HIB), and Helicobacter pylori.

The term "parasitic antigens" according to the invention means such antigens as those derived from organisms causing malaria and Lyme disease.

The term "mammalian antigen" according to the invention means a mammalian protein, peptide, hapten, polysaccharide or lipid, preferably a mammalian protein, which can induce an immune response in rabbits and/or chicken. Preferably the proteins originate from human, mouse, rat, or old world monkey. Preferably the proteins are targets of therapeutic interest, like cytokines and cytokine receptors, Fc receptors, tyrosine kinase receptors, growth factors and growth factor receptors, tumor antigens and their ligands. Especially preferred are human proteins. In a further embodiment of the invention the mammalian proteins are a group of two, three or four proteins, which are homologous proteins of different species (e.g. IGF-1R from human, mouse, cynomolgus and rat).

Preferred examples for growth factors are Adrenomedullin (AM), Angiopoietin (Ang), Autocrine motility factor, Bone morphogenetic proteins (BMPs), Brain-derived neurotrophic factor (BDNF), Epidermal growth factor (EGF), Erythropoietin (EPO), Fibroblast growth factor (FGF), Glial cell line-derived neurotrophic factor (GDNF), Granulocyte colony-stimulating factor (G-CSF), Granulocyte macrophage colony-stimulating factor (GM-CSF), Growth differentiation factor-9 (GDF9), Hepatocyte growth factor (HGF), Hepatoma-derived growth factor (HDGF), Insulin-like growth factor (IGF), Migration-stimulating factor, Myostatin (GDF-8), Nerve growth factor (NGF) and other neurotrophins, Platelet-derived growth factor (PDGF), Thrombopoietin (TPO), Transforming growth factor alpha(TGF-α), Transforming growth factor beta(TGF-(β), Tumor_necrosis_factor-alpha(TNF-α), Vascular endothelial growth factor (VEGF), placental growth factor (PIGF),Foetal Bovine Somatotrophin (FBS), IL-1- Cofactor for IL-3 and IL-6, IL-2-T-cell growth factor, IL-4- Growth factor, IL-5, IL-6, IL-7.

Examples for tyrosine kinase receptors and growth factor receptors are ALK (anaplastic lymphoma kinase), a tyrosine kinase receptor expressed as part of the chimeric NPM-ALK protein, in anaplastic large cell lymphomas (ALCLs); Discoidin domain receptor (DDR), a receptor tyrosine kinase that is distinguished by a unique extracellular domain homologous to the lectin Discoidin I (Discoidin receptor tyrosine kinase) (Tyrosine-protein kinase CAK) (Cell adhesion kinase) (TRK E) (Protein-tyrosine kinase RTK 6) (CD167a antigen); Discoidin domain receptor 2 precursor (Receptor protein-tyrosine kinase TKT) (Tyrosine-protein kinase TYRO 10) (Neurotrophic tyrosine kinase, receptor-related 3); Epidermal growth factor receptor (Receptor protein-tyrosine kinase ErbB-1); Receptor protein-tyrosine kinase erbB-2 (p185erbB2) (NEU proto-oncogene) (C-erbB-2); Receptor protein-tyrosine kinase erbB-3 precursor (c-erbB3) (Tyrosine kinase-type cell surface receptor); Receptor protein-tyrosine kinase erbB-4 (p 180erbB4) (Tyrosine kinase-type cell surface receptor); Basic fibroblast growth factor receptor 1 (FGFR-1) (bFGF-R) (Fms-like tyrosine kinase-2) (c-fgr); FL cytokine receptor (Tyrosine-protein kinase receptor FLT3) (Stem cell tyrosine kinase 1) (STK-1) (CD135 antigen); Mast/stem cell growth factor receptor (SCFR) (Proto-oncogene tyrosine-protein kinase Kit) (c-kit) (CD 117 antigen); Leukocyte tyrosine kinase receptor (Protein tyrosine kinase-1); Hepatocyte growth factor receptor (Met proto-oncogene tyrosine kinase) (c-met) (HGF receptor) (HGF-SF receptor); Protein-tyrosine phosphatase eta (R-PTP-eta) (HPTP eta) (Protein-tyrosine phosphatase receptor type J) (Density enhanced phosphatase-1) (DEP-1) (CD 148 antigen); Proto-oncogene tyrosine-protein kinase receptor ret (C-ret); Tyrosine-protein kinase transmembrane receptor ROR1 (Neurotrophic tyrosine kinase, receptor-related 1); Tyrosine-protein kinase transmembrane receptor ROR2 (Neurotrophic tyrosine kinase, receptor-related 2);Tyrosine-protein kinase receptor Tie-1;Angiopoietin 1 receptor (Tyrosine-protein kinase receptor TIE-2) (Tyrosine-protein kinase receptor TEK) (P140 TEK) (Tunica interna endothelial cell kinase) (CD202b antigen);High affinity nerve growth factor receptor (TRK1 transforming tyrosine kinase protein) (p140-TrkA) (Trk-A);BDNF/NT-3 growth factors receptor (TrkC tyrosine kinase) (GP145-TrkB) (Trk-B); NT-3 growth factor receptor (TrkC tyrosine kinase) (GP145-TrkC) (Trk-C);Vascular endothelial growth factor receptor 1 (VEGFR-1) (Vascular permeability factor receptor) (Tyrosine-protein kinase receptor FLT) (Flt-1) (Tyrosine-protein kinase FRT) (Fms-like tyrosine kinase 1);Vascular endothelial growth factor receptor 2 (VEGFR-2) (Kinase insert domain receptor) (Protein-tyrosine kinase receptor Flk-1); and Vascular endothelial growth factor receptor 3 precursor (EC 2.7.1.112) (VEGFR-3) (Tyrosine-protein kinase receptor FLT4), Insulin Receptor (IR) and Insulin-Like Growth Factor Receptor (IGF-R), Vascular Endothelial Growth Factor Receptor (VEGFR); Platelet-Derived Growth Factor Receptors (PDGFR); C-Kit Receptor (Also Known as the Steel Factor Receptor).

Preferred examples for proteins are also Fibroblast Growth Factor Receptors FGFR1 (CD331), FGFR2 (CD332), FGFR3 (CD333), FGFR4 (CD334), FGFR6.

Preferred examples for Fc Receptors (FcR) are FcγRI (CD64), FcγRIIA (CD32), FcγRIIB1 (CD32), FcγRIIB2 (CD32), FcγRIIIA (CD16a), FcγRIIIB (CD16b), FcεRI, FCαRII (CD23), FcαRI (CD89), Fcα/µR, FcRn.

Preferred examples for cytokines are proteins of the IL-2 subfamily (including erythropoietin (EPO) and thrombopoietin (TPO)), the interferon (IFN) subfamily, the IL-10 subfamily, the IL-1 family (including IL-1 and IL-18), the IL-17 family, preferably IFN-γ, TGF-β, IL-4, IL-10, IL-13,

Preferred examples for cytokine receptors are Cytokine receptor common gamma chain (Gamma-C) (Interleukin-2 receptor gamma chain) (IL-2R gamma chain) (P64) (CD132 antigen); Interleukin-10 receptor alpha chain (IL-10R-A) (IL-10R1); Interleukin-10 receptor beta chain (IL-10R-B) (IL-10R2) (Cytokine receptor class-II CRF2-4); Interleukin-12 receptor beta-1 chain (IL-12R-beta1) (Interleukin-12 receptor beta) (IL-12 receptor beta component) (IL-12RB1); Interleukin-12 receptor beta-2 chain (IL-12 receptor beta-2) (IL-12R-beta2); Interleukin-13 receptor alpha-1 chain (IL-13R-alpha-1) (IL-13RA-1) (CD213a1 antigen); Interleukin-13 receptor alpha-2 chain (Interleukin-13 binding protein); Interleukin-17 receptor (IL-17 receptor); Interleukin-17B receptor (IL-17B receptor) (IL-17 receptor homolog 1) (IL-17Rh1) (IL17Rh1) (Cytokine receptor CRL4) (UNQ2501/PRO19612); Interleukin 21 receptor precursor (IL-21R); Interleukin-1 receptor, type I(IL-1R-1) (IL-1R-alpha) (P80) (Antigen CD121a); Interleukin-1 receptor, type II (IL-1R-2) (IL-1R-beta) (Antigen CDw121b); Interleukin-1 receptor antagonist protein (IL-1ra) (IRAP) (IL1 inhibitor) (IL-1RN) (ICIL-1RA); Interleukin-2 receptor alpha chain (IL-2 receptor alpha subunit) (P55) (TAC antigen) (CD25 antigen); Interleukin-2 receptor beta chain (IL-2 receptor) (P70-75) (High affinity IL-2 receptor beta subunit) (CD122 antigen); Interleukin-3 receptor alpha chain (IL-3R-alpha) (CD 123 antigen); Interleukin-4 receptor alpha chain (IL-4R-alpha) (CD 124 antigen); Interleukin-5 receptor alpha chain (IL-5R-alpha) (CD125 antigen) Interleukin-6 receptor alpha chain (IL-6R-alpha) (IL-6R1) (CD 126 antigen); Interleukin-6 receptor beta chain (IL-6R-beta) (Interleukin 6 signal transducer) (Membrane glycoprotein 130) (gp130) (Oncostatin M receptor) (CDw130) (CD130 antigen); Interleukin-7 receptor alpha chain (IL-7R-alpha) (CDw127) (CD127 antigen); High affinity interleukin-8 receptor A (IL-8R A) (IL-8 receptor type 1) (CXCR-1) (CDw128a); High affinity interleukin-8 receptor B (IL-8R B) (CXCR-2) (GRO/MGSA receptor) (IL-8 receptor type 2) (CDw128b); Interleukin-9 receptor (IL-9R); Interleukin-18 receptor 1 (IL1 receptor-related protein) (IL-1Rrp); Interleukin-1 receptor-like 1 precursor (ST2 protein); Interleukin-1 receptor-like 2 (IL-1Rrp2) (Interieukin-1 receptor related protein 2) (IL1R-rp2); Toll-like receptor 1 (Toll/interleukin-1 receptor-like) (TIL); Toll-like receptor 2 (Toll/interleukin 1 receptor-like protein 4); Toll-like receptor 5 (Toll/interleukin-1 receptor-like protein 3); CX3C chemokine receptor 1 (C-X3-C CKR-1) (CX3CR1) (Fractalkine receptor) (GPR13) (V28) (Beta chemokine receptor-like 1) (CMK-BRL-1) (CMKBLR1); C-X-C chemokine receptor type 3 (CXC-R3) (CXCR-3) (CKR-L2) (CD183 antigen); C-X-C chemokine receptor type 4 (CXC-R4) (CXCR-4) (Stromal cell-derived factor 1 receptor) (SDF-1 receptor) (Fusin) (Leukocyte-derived seven transmembrane domain receptor) (LESTR) (LCR1) (FB22) (NPYRL) (HM89) (CD184 antigen); C-X-C chemokine receptor type 5 (CXC-R5) (CXCR-5) (Burkitt'S lymphoma receptor 1) (Monocyte-derived receptor 15) (MDR15); C-X-C chemokine receptor type 6 (CXC-R6) (CXCR-6) (G protein-coupled receptor bonzo) (G protein-coupled receptor STRL33); Chemokine binding protein 2 (Chemokine-binding protein D6) (C-C chemokine receptor D6) (Chemokine receptor CCR-9) (CC-Chemokine receptor CCR10); C-C chemokine receptor type 1 (C-C CKR-1) (CC-CKR-1) (CCR-1) (CCR1) (Macrophage inflammatory protein-1 alpha receptor) (MIP-1alpha-R) (RANTES-R) (HM145) (LD78 receptor); C-C chemokine receptor type 2 (C-C CKR-2) (CC-CKR-2) (CCR-2) (CCR2) (Monocyte chemoattractant protein 1 receptor) (MCP-1-R); C-C chemokine receptor type 3 (C-C CKR-3) (CC-CKR-3) (CCR-3) (CCR3) (CKR3) (Eosinophil eotaxin receptor); C-C chemokine receptor type 4 (C-C CKR-4) (CC-CKR-4) (CCR-4) (CCR4) (K5-5); C-C chemokine receptor type 5 (C-C CKR-5) (CC-CKR-5) (CCR-5) (CCR5) (HIV-1 fusion coreceptor) (CHEMR13) (CD195 antigen); C-C chemokine receptor type 6 (C-C CKR-6) (CC-CKR-6) (CCR-6) (LARC receptor) (GPR-CY4) (GPRCY4) (Chemokine receptor-like 3) (CKR-L3) (DRY6); C-C chemokine receptor type 7 precursor (C-C CKR-7) (CC-CKR-7) (CCR-7) (MIP-3 beta receptor) (EBV-induced G protein-coupled receptor 1) (EBI1) (BLR2); C-C chemokine receptor type 8 (C-C CKR-8) (CC-CKR-8) (CCR-8) (GPR-CY6) (GPRCY6) (Chemokine receptor-like 1) (CKR-L1) (TER1) (CMKBRL2) (CC-chemokine receptor CHEMR1); C-C chemokine receptor type 9 (C-C CKR-9) (CC-CKR-9) (CCR-9) (GPR-9-6); C-C chemokine receptor type 10 (C-C CKR-10) (CC-CKR-10) (CCR-10) (G-protein coupled receptor 2); C-C chemokine receptor type 11 (C-C CKR-11) (CC-CKR-11) (CCR-11) (Chemokine receptor-like 1) (CCRL1) (CCX CKR); Chemokine receptor-like 1 (G-protein coupled receptor DEZ) (G protein-coupled receptor ChemR23), Chemokine receptor-like 2 (IL8-related receptor DRY12) (Flow-induced endothelial G protein-coupled receptor) (FEG-1) (G protein-coupled receptor GPR30) (GPCR-BR); Chemokine XC receptor 1 (XC chemokine receptor 1) (Lymphotactin receptor) (G protein-coupled receptor 5); Tumor Necrosis Factor Receptors (TNFR).

Preferred examples for tumor antigens are MART-1, carcinoembryonic antigen ("CEA"), gp100, tyrosinase; MAGE-1, HER-2, trp-1, and LewisY antigens, hematopoietic differentiation antigens-glycoproteins usually associated with cluster differentiation (CD) groupings, such as CD5, CD19, CD20, CD22, CD33, CD45, CD52, and CD147; cell surface differentiation antigens, including glycoproteins, such as carcinoembryonic antigen (CEA, Swiss-Prot ID No. P06731), sialyl Tn antigen (TAG-72), polymorphic epithelial mucin (PEM), epithelial cell adhesion molecule (Ep-CAM), MUC-1, A33, G250, E-cadherin, prostate-specific membrane antigen (PSMA, Swiss-Prot ID No. Q04609) and prostate-specific antigen (PSA), glycolipids, such as gangliosides, e.g., GD2, GD3, GM2) and carbohydrates, such as blood group-related antigens, including LEY and LEb (LEY is "LewisY", also known as "CD174"; growth factor receptors, including epidermal growth factor receptor (EGFR, ErbB1, Swiss-Prot ID P00533) and its mutant form EGFRvIII, ErbB2 (HER-2/neu, Swiss-Prot ID No. P04626), ErbB3 (HER-3, Swiss-Prot ID No. P21860) and IL-2 receptor, fibroblast activation protein (FAP), vascular endothelial growth factor receptor (VEGFR), tenascin and integrin; Frizzled receptor family (e.g. Fz-2).

Especially preferred antigens are CEA (Swiss-Prot ID No. P06731), ErbB2 (Swiss-Prot ID No. P04626), EGFR (Swiss-Prot ID No. P00533), LewisY, MUC-1 (Swiss-Prot ID No. P15941), EpCAM (the target of mAb 17-1A (edrecolomab, Panorex^{®}, Glaxo Wellcome GmbH)), CA125 (Swiss-Prot ID No. Q96RK2), PSMA (Swiss-Prot ID No. Q04609), the target of the TAG72 antibody, CD20 (Swiss-Prot ID No. P11836), CD19 (Swiss-Prot ID No. P15391), CD22 (Swiss-Prot ID No. P20273), and CD36 (Swiss-Prot ID No. P16671), ErbB3 (Swiss-Prot ID No. P21860), ErbB4 (Swiss-Prot ID No. Q15303), FGF receptors 1-4 (Swiss-Prot ID Nos. P22455, P11362, P21802, P22607), HGF receptor (Swiss-Prot ID No. P08581), IGF1-R (Swiss-Prot ID No. P08069), Insulin receptor (Swiss-Prot ID No. P06213), PDGF receptors alpha and beta (Swiss-Prot ID Nos. P16234, P09619, respectively) and C-KIT (Swiss-Prot ID No. P10721).

The term "viral or bacterial antigens" refer to components on the surface of viruses or bacteria which induce an immune response in rabbits and/or chicken. As described in WO 2010/101441 an antibody mixture to the F-protein and the G-protein of the RS virus is more effective in neutralizing the virus compared to the standard therapy with the antibodies Synagis and Motavizumab which target only the F-protein. From this a single antibody binding to the two proteins seems to be advantageous over a mixture.

"Antibody fragments" comprise a portion of a full length antibody, preferably the variable regions thereof, or at least the antigen binding site thereof. Examples of antibody fragments include diabodies, Fab fragments, and single-chain antibody molecules. scFv antibodies are, e.g., described in Huston, J.S., Methods in Enzymol. 203 (1991) 46-88.

The terms "monoclonal antibody" or "monoclonal antibody composition" as used herein refer to a preparation of antibody molecules of a single amino acid composition. The term "chimeric antibody" refers to a monoclonal antibody comprising a variable region, i.e., binding region, from rabbit or chicken and at least a portion of a constant region derived from a different source or species, usually prepared by recombinant DNA techniques. According to the invention chimeric antibodies comprising a rabbit or chicken variable region and a human constant region and humanized rabbit or chicken antibodies are especially preferred. Other forms of "chimeric antibodies" encompassed by the present invention are those in which the class or subclass has been modified or changed from that of the original antibody. Such "chimeric" antibodies are also referred to as "class-switched antibodies." Methods for producing chimeric antibodies involve conventional recombinant DNA and gene transfection techniques now well known in the art (see, e.g., Morrison, S.L., et al, Proc. Natl. Acad Sci. USA 81 (1984) 6851-6855; US 5,202,238 and US 5,204,244).

The term "humanized antibody" or "humanized version of an antibody" refers to antibodies in which a human variable region has been modified to comprise the CDRs of an antibody according to the invention. In a preferred embodiment, the CDRs of the VH and VL are grafted into the framework region of human antibody to prepare the "humanized antibody." See e.g. Riechmann, L., et al, Nature 332 (1988) 323-327; and Neuberger, M.S., et al, Nature 314 (1985) 268-270. The heavy and light chain variable framework regions can be derived from the same or different human antibody sequences. The human antibody sequences can be the sequences of naturally occurring human antibodies. Human heavy and light chain variable framework regions are listed e.g. in Lefranc, M.-P., Current Protocols in Immunology (2000) - Appendix IP A.1P.1-A.1P.37 and are accessible via IMGT, the international ImMunoGeneTics information system^{®} (http://imgt.cines.fr) or via http://vbase.mrc-cpe.cam.ac.uk.

The term "recombinant antibody", as used herein, is intended to include all antibodies according to the invention that are prepared by recombinant means, such as antibodies from a host cell such as a NS0 or CHO cell using a recombinant expression vector transfected into a host cell. Such recombinant human antibodies have variable and constant regions in a rearranged form.

The term "antibody which binds specifically to human c-Met and IGF-1R or to c-Met and/or IGF-1R and at least one antigen selected from the group consisting of human EGFR, HER2 and HER3", as used herein, refer to an antibody which binds specifically to the human antigens:
c-Met and IGF-1R,
c-Met and EGFR,
c-Met and HER2,
c-Met and HER3,
c-Met and EGFR and HER2,
c-Met and EGFR and HER3,
c-Met and HER2 and HER3,
c-Met and EGFR, HER2 and HER3
IGF-1R and EGFR,
IGF-1R and HER2,
IGF-1R and HER3,
IGF-1R and EGFR and HER2,
IGF-1R and EGFR and HER3,
IGF-1R and HER2 and HER3,
IGF-1R and EGFR, HER2 and HER3,
c-Met and IGF-1R and EGFR,
c-Met and IGF-1R and HER2,
c-Met and IGF-1R and HER3,
c-Met and IGF-1R and EGFR and HER2,
c-Met and IGF-1R and EGFR and HER3,
c-Met and IGF-1R and HER2 and HER3, or
c-Met and IGF-1R and EGFR, HER2 and HER3.

The term "specifically binding ", as used herein, refer to binding of the antibody to the respective antigen, measured by ELISA, wherein said ELISA comprises coating c-Met and/or IGF-1R, EGFR, HER2, and HER3 protein respectively to a solid support, adding said antibody under conditions to allow the formation of an immune complex with the respective c-Met and/or IGF-1R or HER protein, detecting said immune complex by measuring the Optical Density values (OD) using a secondary antibody binding to an antibody according to the invention and using a peroxidase-mediated color development. Specific binding is found if at an antibody concentration of 300 µmol/l or more an OD value of more than 0.1, 0.3 or more, preferably 1.0 or more, preferably 1.5 or more, preferably 2.0 or more (measured against same solvent without antibody at 450nm) is found. Preferably the antibody according to the invention binds to c-Met and IGF-1R or to c-Met and/or IGF-1R and HER2 in a ratio of 1:10 to 10:1, to c-Met and/or IGF-1R and HER3 in a ratio of 1:10 to 10:1 and to c-Met and/or IGF-1R and EGFR in a ratio of 1:10 to 10:1 measured by ELISA wherein said ELISA comprises: coating c-Met and/or IGF-1R, EGFR, HER2, and HER3 protein respectively to a solid support, adding said antibody under conditions to allow the formation of an immune complex with the respective c-Met and/or IGF-1R or HER protein, detecting said immune complex by measuring the Optical Density using a secondary antibody binding to said antibody and a peroxidase-mediated color development, and calculating said binding ratios using said Optical Density values. The antibodies according to the invention do not bind specifically to other antigens which were not used for immunization. Therefore for these antigens OD values will be 0.1 or lower (preferably 0.09 or lower, 0.08 or lower or even 0.0).

The antibody according to the invention comprises a VH region and a VL region or parts thereof, which are both together sufficient for the specific binding to c-Met and IGF-1R or to c-Met and/or IGF-1R, EGFR, HER2, and/or HER3 according to the invention. Therefore the antibody according to the invention is different to antibodies which are sometimes called "bispecific, multispecific etc. antibodies" in the state of the art and which comprise according to the state of the art at least a VH and a VL region from a first antibody and a VH and a VL region from a second antibody wherein each of those antibodies binds to a different antigen or epitope or which comprise several single-domains of nanobodies (such antibodies of the state of the art are described e.g. by Kontermann R., MAbs. 2012 Mar 1;4(2); and Caravella J, Lugovskoy, A.Curr Opin Chem Biol. 2010 Aug;14(4):520-8).

The terms "EGFR, HER2, and HER3" or "HER antigens" or "HER proteins", as used herein, refer to Human Epidermal growth factor receptor (EGFR), its sequence and functions are described by UniProt P00533 (EC=2.7.10.1), human HER2, its sequence and functions are described by UniProt P04626 (Receptor tyrosine-protein kinase erbB-2, EC=2.7.10.1), and human HER3, its sequence and functions are described by UniProt P21860 (Receptor tyrosine-protein kinase erbB-3, EC=2.7.10.1).

The term "c-Met and/or IGF-1R", as used herein, refers to human c-Met and/or IGF-1R, their sequences and functions are described by UniProt P08581 (Hepatocyte growth factor receptor, EC=2.7.10.1) and UniProt P08069 (IGF-1R).

Amplification of c-Met and/or IGF-1R, EGFR, HER2 and HER3 genes and/or overexpression of its protein have been reported in numerous cancers, including prostate, bladder, and breast tumors.

The "variable region (or domain) of an antibody according to the invention" (variable region of a light chain (VL), variable region of a heavy chain (VH)) as used herein denotes each of the pair of light and heavy chain regions which are involved directly in binding the antibody to the antigen. The variable light and heavy chain regions have the same general structure and each region comprises four framework (FR) regions whose sequences are widely conserved, connected by three complementary determining regions, CDRs. The term "antigen-binding portion of an antibody" when used herein refer to the amino acid residues of an antibody which are responsible for antigen-binding. The antigen-binding portion of an antibody comprises preferably amino acid residues from the "complementary determining regions" or "CDRs". The CDR sequences are defined according to Kabat et al, Sequences of Proteins of Immunological Interest, 5th Ed. Public Health Service, National Institutes of Health, Bethesda, Md. (1991). Using this numbering system, the actual linear amino acid sequence may contain fewer or additional amino acids corresponding to a shortening of, or insertion into, a FR or CDR of the variable region. For example, a heavy chain variable region may include a single amino acid insert (residue 52a according to Kabat) after residue 52 of H2 and inserted residues (e.g. residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

In one embodiment the antibody according to the invention comprises an Fc part derived from human origin and preferably all other parts of the human constant regions. As used herein the term "Fc part derived from human origin" denotes a Fc part which is either a Fc part of a human antibody of the subclass IgG1, IgG2, IgG3 or IgG4, e.g. a Fc part from human IgG1 subclass, a mutated Fc part from human IgG1 subclass (preferably with a mutation on L234A + L235A), a Fc part from human IgG4 subclass or a mutated Fc part from human IgG4 subclass (preferably with a mutation on S228P). In one embodiment the antibody according to the invention is of human IgG1 subclass. Human constant chains are well known in the state of the art and e.g. described by Kabat, E.A., (see e.g. Johnson, G. and Wu, T.T., Nucleic Acids Res. 28 (2000) 214-218).

Cell-mediated effector functions like ADCC of antibodies according to the invention can be enhanced by engineering their oligosaccharide component as described in Umana, P., et al, Nature Biotechnol. 17 (1999) 176-180, and US 6,602,684, WO 2005/044859, WO 2004/065540, WO2007/031875. The term "antibody-dependent cellular cytotoxicity (ADCC)" refers to lysis of human target cells by an antibody according to the invention in the presence of effector cells.

The antibodies according to the invention are preferably produced by recombinant means. Such methods are widely known in the state of the art and comprise protein expression in prokaryotic and eukaryotic cells with subsequent isolation of the antibody polypeptide and usually purification to a pharmaceutically acceptable purity. For the protein expression nucleic acids encoding light and heavy chains of an antibody according to the invention or fragments thereof are inserted into expression vectors by standard methods. Expression is performed in appropriate prokaryotic or eukaryotic host cells, such as CHO cells, NSO cells, SP2/0 cells, HEK293 cells, COS cells, yeast, or E. coli cells, and the antibody is recovered from the cells (from the supernatant or after cells lysis). Recombinant production of antibodies is well-known in the state of the art and described, for example, in the review articles of Makrides, S.C., Protein Expr. Purif. 17 (1999) 183-202; Geisse, S., et al, Protein Expr. Purif. 8 (1996) 271-282; Kaufman, R.J., Mol. Biotechnol. 16 (2000) 151-161; Werner, R.G., Drug Res. 48 (1998) 870-880. The antibodies may be present in whole cells, in a cell lysate, or in a partially purified, or pure form. Purification is performed in order to eliminate other cellular components or other contaminants, e.g., other cellular nucleic acids or proteins, by standard techniques, including, column chromatography and others well known in the art (see Ausubel, F., et al, ed. Current Protocols in Molecular Biology, Greene Publishing and Wiley Interscience, New York (1987)). Expression in NSO cells is described by, e.g., Barnes, L.M., et al, Cytotechnology 32 (2000) 109-123; Barnes, L.M., et al, Biotech. Bioeng. 73 (2001) 261-270. Transient expression is described by, e.g.,Durocher, Y., et al, Nucl. Acids. Res. 30 (2002) E9. Cloning of variable domains is described by Orlandi, R., et al, Proc. Natl. Acad. Sci. USA 86 (1989) 3833- 3837; Carter, P., et al, Proc. Natl. Acad. Sci. USA 89 (1992) 4285-4289; Norderhaug, L., et al, J. Immunol. Methods 204 (1997) 77-87. A preferred transient expression system (HEK 293) is described by Schlaeger, E.-J. and Christensen, K., in Cytotechnology 30 (1999) 71-83, and by Schlaeger, E.-J., in J. Immunol. Methods 194 (1996) 191-199. Monoclonal antibodies are suitably separated from the culture medium by conventional immunoglobulin purification procedures such as, for example, protein A-Sepharose, hydroxylapatite chromatography or affinity chromatography.

DNA and RNA encoding the monoclonal antibodies are sequenced using conventional procedures. RT PCR is preferably used.

Antibodies obtained from said cell lines are preferred embodiments of the invention. Amino acid sequence variants of an antibody are prepared by introducing nucleotide changes into the antibody encoding DNA, or by peptide synthesis. Any cysteine residue not involved in maintaining the proper conformation of the antibody may also be substituted, generally with serine, to improve the oxidative stability of the molecule and to prevent aberrant crosslinking. Conversely, cysteine bond(s) may be added to the antibody to improve its stability (particularly where the antibody is an antibody fragment such as an Fv fragment).

The heavy and light chain variable regions according to the invention are combined with sequences of promoter, translation initiation, constant region, 3' untranslated region, polyadenylation, and transcription termination to form expression vector constructs. The heavy and light chain expression constructs can be combined into a single vector, co-transfected, serially transfected, or separately transfected into host cells which are then fused to form a single host cell expressing both chains.

The term "respectively" means that for production (immunization) of a bispecific antibody according to the invention at least the respective two antigens must be used, for production of a trispecific antibody according to the invention at least the respective three antigens must be used, for the production of a tetraspecific antibody according to the invention at least the respective four antigens must be used, and for the production of a pentaspecific antibody according to the invention at least the respective five antigens must be used. A bispecific antibody against EGFR and HER2 obtainable by immunization with EGFR or HER2 alone is therefore preferably excluded from the invention. Therefore and for example, an antibody specifically binding to c-Met and IGF-1R or to c-Met and/or IGF-1R and HER2 is selected and produced if immunization is performed with at least the two antigens c-Met and/or IGF-1R and HER2. An antibody specifically binding to c-Met and IGF-1R or to c-Met and/or IGF-1R, EGFR and HER3 is selected and produced if immunization is performed with at least the respective antigens c-Met, IGF-1R, EGFR and HER3. An antibody specifically binding to c-Met and/or IGF-1R, EGFR, HER2 and HER3 is selected and produced if immunization is performed with the four or five antigens c-Met and/or IGF-1R, EGFR, HER2 and HER3. Preferably for immunization all four or five antigens are used.

One aspect of the invention is a pharmaceutical composition comprising an antibody according to the invention. Another aspect of the invention is the use of an antibody according to the invention for the manufacture of a pharmaceutical composition. A further aspect of the invention is a method for the manufacture of a pharmaceutical composition comprising an antibody according to the invention. In another aspect, the present invention provides a composition, e.g. a pharmaceutical composition, containing an antibody according to the present invention, formulated together with a pharmaceutical carrier.

Furthermore the antibodies according to the invention are especially useful for the treatment of cancer. Therefore one aspect of the invention is a pharmaceutical composition for the treatment of cancer.

Another aspect of the invention is an antibody according to the invention for the treatment of cancer. For this the antibody according to the invention can be investigated in a respective mouse tumor model e.g. according to Krupke DM; Begley DA; Sundberg JP; Bult CJ; Eppig JT, The Mouse Tumor Biology database., Nat Rev Cancer 2008 Jun;8(6):459-65. Therefore one aspect of the invention is a pharmaceutical composition for the treatment of cancer.

Another aspect of the invention is the use of an antibody according to the invention for the manufacture of a medicament for the treatment of cancer.

Another aspect of the invention is a method of treatment of a patient suffering from cancer by administering an antibody according to the invention to said patient in the need of such treatment.

As used herein, "pharmaceutical carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (e.g. by injection or infusion).

A composition of the present invention can be administered by a variety of methods known in the art. As will be appreciated by the skilled artisan, the route and/or mode of administration will vary depending upon the desired results. To administer a compound of the invention by certain routes of administration, it may be necessary to coat the compound with, or co-administer the compound with, a material to prevent its inactivation. For example, the compound may be administered to a subject in an appropriate carrier, for example, liposomes, or a diluent. Pharmaceutically acceptable diluents include saline and aqueous buffer solutions. Pharmaceutical carriers include sterile aqueous solutions or dispersions and sterile powders for the extemporaneous preparation of sterile injectable solutions or dispersion. The use of such media and agents for pharmaceutically active substances is known in the art.

The phrases "parenteral administration" and "administered parenterally" as used herein means modes of administration other than enteral and topical administration, usually by injection, and includes, without limitation, intravenous, intramuscular, intra-arterial, intrathecal, intracapsular, intraorbital, intracardiac, intradermal, intraperitoneal, transtracheal, subcutaneous, subcuticular, intra-articular, subcapsular, subarachnoid, intraspinal, epidural and intrasternal injection and infusion.

The term "cancer" as used herein may be, for example, lung cancer, non-small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma, lymphoma, lymphocytic leukemia, including refractory versions of any of the above cancers, or a combination of one or more of the above cancers. Preferably such cancer is a breast cancer, colon cancer, lung cancer, or pancreatic cancer. Preferably such cancers are further characterized by c-Met, IGF-1R, EGFR, HER2 and/or HER3 expression or overexpression.

These compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol, sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin. Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art. Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The method according to the invention comprises in summary the steps of immunization, B cell isolation, enrichment of B cells, isolation of single B cells, preferably co-cultivation with feeder cells, selection of a single B cell which comprises respective mRNA, and production of the antibody according to the invention. Such methods are mentioned for the production of monospecific antibodies e.g. in WO2011147903, WO2007003041, WO2008045140, WO2004106377, EP1255780, and EP1633787.

### Immunization

Immunization can be performed according to the methods known of the state of the art, e.g. by using DNA of the target antigens or fragments thereof, complete protein antigens or fragments thereof, antigen expressing cells. Preferably the target antigens, preferably c-Met, IGF-1R, EGFR, HER2 and/or HER3 antigen is/are fusion polypeptides consisting of said antigen and a human Fc polypeptide. Preferably immunization in step i) is repeated at least three times and appropriately up to six times during 90 days (if an antibody according to the invention is identified already after e.g. the fourth immunization, further immunizations are not necessary). Preferably complete Freund's adjuvant (CFA) or CFA and incomplete Freund's adjuvant (IFA) is (are) used as adjuvant.

### B cell isolation

The B-cells are isolated from the rabbit or chicken, preferably from the blood of the rabbit or chicken. The B-cells are isolated up to 8 days, preferably 6 to 8 days, after 3rd to 6th immunization. Preferably PBMCs are isolated and depleted from macrophages (see e.g. EP0488470) and used as B cells in step iii). Isolation of B cells can be for example also performed by labeling non-B cells with non B cell markers, e.g. anti CD2, CD14, CD16, CD36, CD43, and CD235a antibodies and separating the labeled non B cells from non-labeled B cells.

### Enrichment of B cells

Antigen specific B cells are preferably isolated (enriched) in step iii) by treating the B cells with one or more target antigens used for immunization, preferably c-Met, IGF-1R, EGFR, HER2 and/or HER3 or a cell expressing one or more of the respective antigens. The antigens used for enrichment must not be all antigens used for immunization. It is also sufficient to use in this step at least of the antigens. Preferably the antigens and the cell expressing the antigens are used in immobilized manner, so that the antigen specific B cells can be separated easily. Such methods are e.g. described in Kodituwakko AP et al., Immunol. Cell Biol. (2003) 81,163-170 and EP0488470.

### Isolation of single B cells

Isolation of single rabbit or chicken B cells in step iv) is preferably performed by FACS. Preferably an anti-rabbit IgG, or an anti-chicken IgG, respectively, is used for FACS selection. Such selected single B cells are antibody producing B cells.

### Co-cultivation with feeder cells

Preferably the antigen producing B cells are co-cultivated with feeder cells after step iv) and before the selection step v) is performed. This increases the amount of antibody in the cell supernant and facilitates analysis and selection of secreted rabbit antibodies specifically binding to the antigens used for immunization, preferably to human c-Met, IGF-1R, EGFR, HER2 and/or HER3 respectively (see e.g. Zubler, R.H., et al., Eur. J. Immunol. 14 (1984) 357-63, Wen L. et al., Eur. J. Immunol. 17 (1987) 887-92, Hoffmann P et al., J Immunol. Methods 1996;196(1):85-91, Roy A. et al., J Hematother. Stem Cell Res. 2001; 10(6):873-80, Dlu A. et al., Proc. Nati. Acad. Sci. USA Vol. 84, pp. 9140-9144, 1987, and EP0488470).

### Selection of a single B cell which comprises mRNA

Selection of a single B cell which comprises mRNA encoding polypeptides comprising a heavy and light chain variable region of an antibody according to the invention can be performed, preferably after co-cultivated with feeder cells, by analyzing the cell supernatant for secreted rabbit or chicken antibodies specifically binding to the antigens used for immunization, preferably to human c-Met, IGF-1R, EGFR, HER2 and/or HER3 respectively. Analysis is preferably performed by ELISA. Immunoglobulin sequences can be then recovered from the selected single human B cell e.g. according to de Wildt RM, Hoet RM. Methods Mol. Biol. 2002; 178:121-31 and analyzed e.g. by RT PCR.

The production of an antibody according to the invention, expressed by a single B cell, can be performed by recombinant means.

Techniques and procedures described or referenced herein are for example, the widely utilized methodologies described in Sambrook et al., Molecular Cloning: A Laboratory Manual 3rd. edition (2001) Cold Spring Harbor Laboratory Press, Cold Spring Harbor, N.Y. CURRENT PROTOCOLS IN MOLECULAR BIOLOGY (F. M. Ausubel, et al. eds., (2003)); the series METHODS IN ENZYMOLOGY (Academic Press, Inc.): PCR 2: A PRACTICAL APPROACH (M. J. MacPherson, B. D. Hames and G. R. Taylor eds. (1995)), Harlow and Lane, eds. (1988) ANTIBODIES, A LABORATORY MANUAL, and ANIMAL CELL CULTURE (R. I. Freshney, ed. (1987)).

The following examples and sequences are provided to aid the understanding of the present invention. It is understood that modifications can be made in the procedures set forth without departing from the spirit of the invention.

### Examples

### Example 1

### Immunization of rabbits (scheme 1)

Five recombinant human Fc-chimera proteins fused with the extracellular domains of c-Met and IGF-1R or to c-Met and/or IGF-1R and the tyrosine kinase receptors Her1, Her2 and Her3 were used as immunogen mixture. Two different immunization schemes, scheme 1 and scheme 2 were explored.

For the immunization according to scheme 1, three New Zealand White (NZW) rabbits were immunized by injecting 1ml of immunogen mixture in each of the animals at day 0, 7, 14, 28, 56 and 84. Proteins were diluted in PBS, pooled in equimolar amounts and mixed 1:1 (v/v) with complete Freund's adjuvant (CFA) before use. A final concentration of 100µg of each immunogen (300µg in total) was used per animal for the 1st immunization and for the 2nd, 3rd, 4th, 5th and 6th immunization 50µg of each immunogen (150µg in total) and per animal was used. Blood samples were collected in tubes, coated with EDTA, five, six and seven days post-immunization after the 3rd, 4th, 5th and 6th Immunization.

### Example 2

### Immunization of rabbits (scheme 2)

For the immunization according to scheme 2, each of the three NZW rabbits were immunized subcutaneously with 1ml of immunogen mixture at day 0, 7, 14, 28, 42 and 56. For the first injection, proteins were diluted in PBS, pooled in equimolar amounts and mixed 1:1 (v/v) with CFA before use. A final concentration of 100µg of each Immunogen (300µg in total) per animal was used for the 1st immunization. For the 2nd ,3rd, 4th, 5th and 6th immunization, proteins were diluted in PBS, pooled in equimolar amounts and mixed 1:1 (v/v) with incomplete Freund's adjuvant (IFA) before use. 50µg of each Immunogen (150µg in total) was used per animal. Blood samples were collected in tubes, coated with EDTA, five, six and seven days post-immunization after the 3rd, 4th, 5th and 6th Immunization at intervals of 2 weeks.

### Example 3

### 3.1 Immunogen Coating/Cell Preparation

The five fusion-proteins used for immunization were coated onto a surface of a cell-culture 6-well plate with a concentration of 8µg in PBS/10cm² and incubated. Alternatively plates were seeded with a cell line BT-474 (DSMZ ACC 64) expressing the three tyrosine kinase receptors EGFR, Her2 and Her3 on their cell surface. One day before use cells were seeded in DMEM+5%FCS at a density leading to about 90% confluence after 24h.

### 3.2 Isolation of peripheral blood mononuclear cells from rabbits

PBMCs were isolated from whole blood of immunized rabbits. The blood was diluted 1:1 with PBS and layered on Lympholyte^{®} according to the manufacturer's instructions (Cedarlane, CL5120). Peripheral blood mononuclear cells (PBMC) were separated from erythrocytes by density gradient centrifugation (800xg, 20min, RT). Cells were removed from the interface, washed twice with PBS (800xg, 10min) and suspended in RPMI 1640 based cell culture medium.

### 3.3 Monocyte depletion

PBMCs were incubated in cell culture medium on plastic. Unbound lymphocytes were collected after incubation time.

### 3.4 Enrichment of antigen specific cells

Antigen specific lymphocytes were enriched on Immunogen coated microtiter plates or directly on BT-474 cells (see 3.1.). Lymphocytes were washed twice with PBS to remove unspecific cells and subsequently incubated with 750µl Trypsin per 10cm² culture surface for 7-10min. Detached cells were collected in cell culture medium for further steps.

### 3.5 Single-cell sorting of Immunoglobulin G-secreting lymphocytes

PBMCs/lymphocytes were stained with a FITC (Fluorescein Isothiocyanate Isomer 1) conjugated goat anti-rabbit IgG antibody, Abd Serotec, STAR121F). A flow cytometric analysis and single-cell sorting was performed with a FACS cytometer. Single positive lymphocytes were sorted directly to 200µl cell culture medium covering 3,0x10⁶ irradiated EL-4 B5 feeder cells (L. Wen et al. Eur. J. Immunol. 17 (1987) 887-892). The cell culture medium described above was supplemented with 5% activated T-cell macrophage supernatant from rabbits (Microcoat). Co-cultivation medium was supplemented with 2x10⁻⁶ g/ml SAC (Staphylococcus Aureus Cowan) solution. After co-cultivation of B-cells and feeder cells for 7 days supernatants were transferred for antibody detection and cells were harvested in 100µl RNA isolation buffer (Qiagen, RLT).

### 3.6 Screening for Immunoglobulin's via enzyme-linked immunosorbent assay

Secreted rabbit antibodies were detected by analyzing the supernatant via a biotinylated capturing antibody (anti-rabbit IgG antibody produced in goat) with a final concentration of 1 µg/ml PBS+0,5%BSA+0,05%Tween^{®}20, coated on streptavidin microtiter plates and a horse radish peroxidase coupled anti-rabbit IgG detection antibody with a final concentration of 1:7500. Washing steps were performed by using PBS+0.1%Tween^{®}20. 3,3',5,5'-Tetramethylbenzidine (TMB) was used as substrate and HCl to stop the enzymatic reaction.

### 3.7 Determination of c-Met and/or IGF-1R and HER specific antibodies in B-cell Supernatants

Microtiter plates were coated with c-Met, IGF-1R, EGFR, HER2, and/or HER3 protein (recombinant Fc chimeric conjugates of human c-Met, IGF-1R, EGFR, HER2, HER3 or IL12 Rß1). After a blocking process, specific antibodies from B-cell supernatants bind to the targets and are then detected by a POD-labeled anti-rabbit IgG antibody. The IL12R binding was used as a counterscreen. The c-Met and/or IGF-1R and HER proteins were tagged with a linker, huFc and His (HER1 does not have a His-tag) like the IL12R protein. Antibodies which bind to the tag were positive in both assays, whereas antigen specific antibodies just bound to the HER proteins and not to IL12R.

12.5µL 0.5µg/mL protein in PBS was transferred to a microtiter plate, incubated and washed 3x with Wash Buffer. 90µL Block Buffer was added to each well, incubated and washed. 12.5µL Standard Antibody (Anti-EGF-Receptor; HER2 Rabbit anti-Human Monoclonal (extracellular domain) (SP3) Antibody; HER3 (N-terminal) antibody; start concentration 2µg/ml; IL-12Rbeta1 antibody start concentration 500ng/ml) in 1:2 dilutions or sample diluted in ELISA buffer was added, incubated and washed. 12.5µl 1:5000 POD-Antibody (Anti-rabbit IgG, peroxidase-linked species-specific Fab2 fragment (from donkey) (ECL); assay dilution: 1:5000) in Elisa Buffer was added, incubated and washed. 15µl TMB was added and 15µl HCl was added after sufficient development. Absorbance (Optical Density O.D.) was read at 450nm/620nm. Results are shown in table 1.

ELISA Buffer: PBS, 0.5% BSA, 0.05% Tween^{®}20
Wash Buffer: PBS, 0.1% Tween^{®}20
Block Buffer: PBS, 2% BSA, 0.05% Tween^{®}20

**Table 1**

| **Mab ID** | **HER1** | **HER2** | **HER3** | **c-Met** | **IGF1R** | **IL12R** | **IgG [ng/mL]** |
|---|---|---|---|---|---|---|---|
| B02 | 0,0019 | -0,0002 | -0,0017 | 0,1994 | 0,2691 | -0,0006 | 1907,50 |
| B08 | 0,4049 | 0,4021 | 1,1432 | 0,7001 | -0,0006 | 0,0294 | 183,28 |
| D02 | 0,2621 | 0,2393 | 0.4499 | 0,3548 | 0,0042 | 0,0490 | 36,727 |
| H04 | 0,2027 | 0,0016 | 2,9544 | 0,0045 | 0,0059 | 0,0060 | 1028,10 |
| D03 | 0,3768 | 0,0120 | 0,5077 | 0,0011 | 0,0027 | 0,0034 | 41,13 |
| A08 | 3,2399 | 0,0258 | 0,3215 | -0,0026 | -0,0028 | 0,0050 | 448,98 |
| H08 | 0,2321 | 0,0085 | 2,1064 | 0,0199 | -0,0021 | 0,0305 | 33,92 |
| B12 | 2,8652 | 0,0335 | 2,5393 | 0,0039 | -0,0003 | 0,0067 | 350,22 |
| D10 | 1,2243 | 0,0014 | 0,9279 | 0,0025 | -0,0067 | 0,0045 | 0 |
| B12 | 0,1119 | 0,0011 | 0,2811 | 0,0027 | 0,0008 | -0,0054 | 224,44 |
| D10 | 0,1379 | -0,0012 | 0,1670 | 0,0002 | -0,0054 | -0,0050 | 101,31 |
| F07 | 0,1080 | -0,0005 | 3,3611 | -0,0003 | -0,0029 | -0,0013 | 691,45 |
| B08 | 0,0636 | 0,1119 | 0,2419 | 0,1436 | -0,0007 | 0,02405 | 51,60 |
| D11 | 0,0608 | 0,1189 | 1,7568 | 0,1494 | 0,0023 | 0,0165 | 69,04 |
| C05 | 0,0004 | 1,1818 | 0,1165 | -0,0017 | 0,001 | 0,0113 | 629,03 |
| B11 | 0,0753 | 0,1464 | 2,5021 | 0,0629 | 0,0029 | 0,0540 | 0 |
| D08 | -0,0005 | 0,0004 | 0,7432 | 2,1724 | 0,0030 | 0,0017 | 102,34 |
| D03 | 0,0001 | 0,0003 | 0,304 | 0,0015 | 0,6134 | 0,0017 | 124,23 |
| A05 | 0,0002 | 0,1563 | -0,0034 | 0,7835 | -0,0012 | -0,0013 | 1101,50 |
| G04 | 0,0743 | 0,1912 | 0,0889 | 0,1559 | -0,001 | 0,0469 | 217,44 |
| B04 | 1,4021 | 3,1074 | 0,0013 | -0,0020 | 0,08295 | 0,0037 | 160,08 |
| B03 | 0,2157 | 0,7911 | -0,0029 | -0,0003 | -0,0033 | 0,0019 | 0 |

| | | | | | | | |
|---|---|---|---|---|---|---|---|
| Underlined values mean specific binding of the antibody to the antigen | | | | | | | |

### Example 4

### Immunization of rats (comparison)

Immunization was performed according to example 1 (immunization scheme 1) with Wistar rats and EGFR, HER2 and HER3 antigens.

Overall 441 anti-huHer1, 774 anti-huHer2 and 299 anti-huHer3 antibodies were isolated after four blood sample collection steps from each rat (10 weeks after immunization start) by a multiple immunization strategy. No multispecific antibodies were identified.

## Claims

1. A monoclonal antibody, **characterized in** specifically binding to three, four or five antigens selected from the group of target antigens.

2. An antibody according to claim 1, **characterized in** specifically binding to human c-Met and IGF-1R or to c-Met and/or IGF-1R and at least one antigen selected from the group consisting of EGFR, HER2 and HER3.

3. An antibody according to claim 1 or 2, **characterized in** being a monoclonal rabbit or chicken antibody.

4. Pharmaceutical composition **characterized by** comprising an antibody according to any one of claims 1 to 3.

5. An antibody according to any one of claims 1 to 3 for use in the treatment of cancer.

6. A method for the production of a monoclonal antibody specifically binding to two, three, four or five antigens selected from the group consisting of target antigens, **characterized in** performing in the order specified the steps of
i) immunizing a rabbit or chicken with two, three, four or five said antigens,
ii) isolating B cells from said rabbit or chicken,
iii) isolating B cells from said B cells isolated in step ii) which bind at least to one of said antigens,
iv) isolating single B cells from said B cells isolated in step iii),
v) selecting one of said single B cells which comprises mRNA encoding a polypeptide, comprising a VH region and/or a polypeptide comprising a VL region of an antibody which binds specifically to said two, three, four or five antigens, and
vi) producing the antibody expressed by said single B cell.

7. A method according to claim 6, **characterized in** the monoclonal antibody is a monoclonal antibody specifically binding to c-Met and IGF-1R or c-Met and/or IGF-1R and at least one antigen selected from the group consisting of EGFR, HER2 and HER3, and the method is **characterized in** performing in the order specified the steps of
i) immunizing a rabbit or chicken with antigens c-Met and IGF-1R or c-Met and/or IGF-1R and at least one antigen selected from the group consisting of EGFR, HER2 and HER3,
ii) isolating B cells from said rabbit or chicken,
iii) isolating B cells from said B cells isolated in step ii) which bind to human c-Met, IGF-1R, EGFR, HER2 and/or HER3,
iv) isolating single B cells from said B cells isolated in step iii),
v) selecting one of said single B cells which comprises mRNA encoding a polypeptide comprising a VH region and/or a polypeptide comprising a VL region of an antibody which binds specifically to c-Met and IGF-1R or to c-Met and/or IGF-1R and at least one antigen selected from the group consisting of EGFR, HER2 and HER3, and
vi) producing the antibody expressed by said single B cell.

8. A rabbit or chicken B cell, **characterized in** comprising mRNA encoding a polypeptide comprising a VH region and/or mRNA encoding a polypeptide comprising a VL region of an antibody according to any one of claims 1 to 3.

9. A method for the production of an antibody according to any one of claims 1 to 3, **characterized in** isolating from a rabbit or chicken B cell comprising mRNA encoding a polypeptide comprising a VH region and/or mRNA encoding a polypeptide comprising a VL region of said antibody, expressing recombinantly said antibody and selecting said antibody if it binds specifically to three, four or five antigens selected from the group consisting of target antigens.

10. A method for the production of an antibody according to any one of claims 1 to 3, **characterized in** isolating from a rabbit or chicken B cell comprising mRNA encoding a polypeptide comprising a VH region and/or mRNA encoding a polypeptide comprising a VL region of said antibody, expressing recombinantly said antibody and selecting said antibody if it binds specifically to c-Met and IGF-1R or to c-Met and/or IGF-1R and at least one antigen selected from the group consisting of EGFR, HER2 and HER3.
